# EUROPEAN PATENT SPECIFICATION

(11) **EP 0 971 646 B1**
(45) Date of publication and mention of the grant of the patent: **22.09.2004**
(21) Application number: 98918112.8
(22) Date of filing: 09.04.1998
(51) Int. Cl.: A61F 2/06

(54) **ENDOVASCULAR GRAFT FOR REPAIRING ABDOMINAL AORTIC ANEURYSMS**
ENDOVASKULÄRES IMPLANTAT ZUM AUSBESSERN EINES ANEURYSMAS DER ABDOMINALAORTA
PROTHESE ENDOVASCULAIRE POUR LA REPARATION DES ANEVRISMES DE L'AORTE ABDOMINALE

(30) Priority: 10.04.1997 GR 97100134
(43) Date of publication of application: 19.01.2000
(73) Proprietor: WILLIAM COOK, EUROPE A/S, 4632 Bjaeverskov (DK); Cook Incorporated, Bloomington, Indiana 47402-0489 (US)
(72) Inventor: PAPAZOGLOU, Konstantinos, GR-552 36 Thessaloniki (GR); DEBRUYNE, Michael, P., Bloomington, IN 47408 (US); DEFORD, John, A., Bloomington, IN 47408 (US); RASMUSSEN, Erik, E., DK-4200 Slagelse (DK)
(74) Representative: Johnston, Kenneth Graham
(86) International application number: PCT/US1998/007303
(87) International publication number: WO 1998/044873

(56) References cited:
- EP-A- 0 686 379
- EP-A- 0 722 701
- EP-A- 0 765 643
- WO-A-95/13033
- WO-A-95/21592
- WO-A-97/09008
- DE-U- 9 319 267

## Description

The present invention relates to grafts for repairing aortic aneurysms.

The endovascular approach to aneurysms of the abdominal aorta is a new technique for the therapy of aneurysms by the placement of grafts which are transferred to the position of placement by means of the vascular lumen from easily anatomically approachable regions, thus avoiding the need for massive surgical operations. For the effective therapy of an aneurysm by this technique, it is necessary to have good circumferential application (contact) of the endovascular graft with the healthy, nondistended part of the blood vessel, so as to have complete exclusion of the distended arterial lumen by the pressure of the systemic arterial circulation, since the blood will now come out through the graft which substitutes for the vascular lumen. Furthermore, the endovascular graft must have a small starting-off diameter, which will allow its easy introduction and advancement from the entrance blood vessel to the position of placement, as well as its easy technical positioning. In abdominal aortic aneurysms there usually is a central neck region of the healthy blood vessel having a normal diameter underneath the kidney arteries (at the point where the graft is surgically attached even by the classical operation). However, often the aortic distention is peripherally extended to the point of the aortic bifurcation at the two iliac arteries. This fact excludes the possibility of placing an endovascular tube graft due to the absence of a healthy peripheral contact point (neck). This often creates the necessity for the placement of endovascular grafts which can be attached to healthy regions of the iliac arteries more peripherally positioned to the distended aortic bifurcation with the simultaneous branching of the aortic blood flow to two cylinders of effluence. The systems that till now have been presented for the placement of aortic stent grafts are composed , first off, of a stent graft or of grafts comprised of two parts that are often complicated in their placement, have a large compressed size, and have imperfections in their support mechanisms and at their point of contact with the vascular wall. These result in the appearance of immediate or future complications or in the inability of placement for a sufficient number of circumstances.

Furthermore, it is a device that, when combined with the fact that it has a limited number of parts, can serve a large number of circumstances of different anatomical dimensions.

A bifurcated endoluminal prosthesis is described in WO-A-95 21 592.

According to the present invention, there is provided a graft arrangement for repairing an aortic aneurysm, the arrangement comprising a main graft, to be endovascularly introduced into the aorta via, for example, an iliac artery, the main graft being expandable and having a proximal orifice to be located in a part of the aorta adjacent to the renal arteries, the main graft also having a distal orifice end which when expanded serves to receive the proximal end(s) of at least one or a pair of expandable iliac artery grafts, wherein each graft comprises an expandable stent and at least one cover over and/or in the start, and wherein the cross-sectional area of the said distal orifice when expanded is sufficiently less than the at least one cross-sectional area or the sum of the cross-sectional areas of the proximal ends of the iliac artery grafts when expanded so as to form a seal with the said distal orifice when the at least one or the pair of grafts are expanded therein. The graft arrangement includes an additional graft endovascularly inserted into the part of the aorta adjacent to the renal arteries, wherein the additional graft has, when expanded, a distal orifice region of cross-sectional area(s) less than that of the proximal orifice region so as to provide reinforcement and support for the proximal end of the main graft. The additional graft including an expandable stent and at least one cover over and/or in the stent. Furthermore, the stent(s) of the additional part includes spikes, barbs, or hooks to facilitate securement to the part of the aorta adjacent to renal arteries. By way of example, the reinforcement is provided by folds in an internally folded cover to provide an increased fitness of cover material. The external cover has a proximal extension on the outer member of each seal whereby the extension is designed folded over the proximal end of the outer member and entering the proximal end of the internal member of each seal. As a result, such folded-over cover provides an extra sealing facility.

The distal end of the main graft can have a part which is extended to form an iliac artery graft. Another part of the main graft has a distal opening or orifice which has a short inclined extension subtended in a distal direction so as to enable an iliac artery graft to be located therein when the short extension has been expanded. Such iliac artery graft has a proximal end which when expanded forms a seal with the short extension.

The distal end of the main graft can have first and second or a pair of short extensions. One extension having the at least one distal orifice of the main graft, and the other extension having another distal orifice for the receipt of a respective iliac artery graft. Each of the iliac artery grafts will have a stent expandable to a cross-sectional area sufficiently greater than the cross-sectional area(s) of the distal orifices so that effective seals are formed.

The main graft can be selected to be of a length to extend from the said part to the bifurcation region wherein the aorta extends into the iliac arteries. Alternatively, the main graft can be of a length such that it extends only across the said part. The part of the main graft in the region of the distal orifice is reinforced in order to support the iliac artery grafts when expanded. It is preferred for these stents to be self expanding. The cover at the distal end of the main graft preferably extends beyond the distal orifice so that after the iliac grafts have been inserted and expanded, the extension to the cover enters the interior of the iliac grafts and forms an extra seal therewith. The start of the main graft can comprise spikes, hooks, or barbs designed to enter the wall of the said part in order to assist in the attachment of that graft to the said part.

### Brief Description of the Drawings

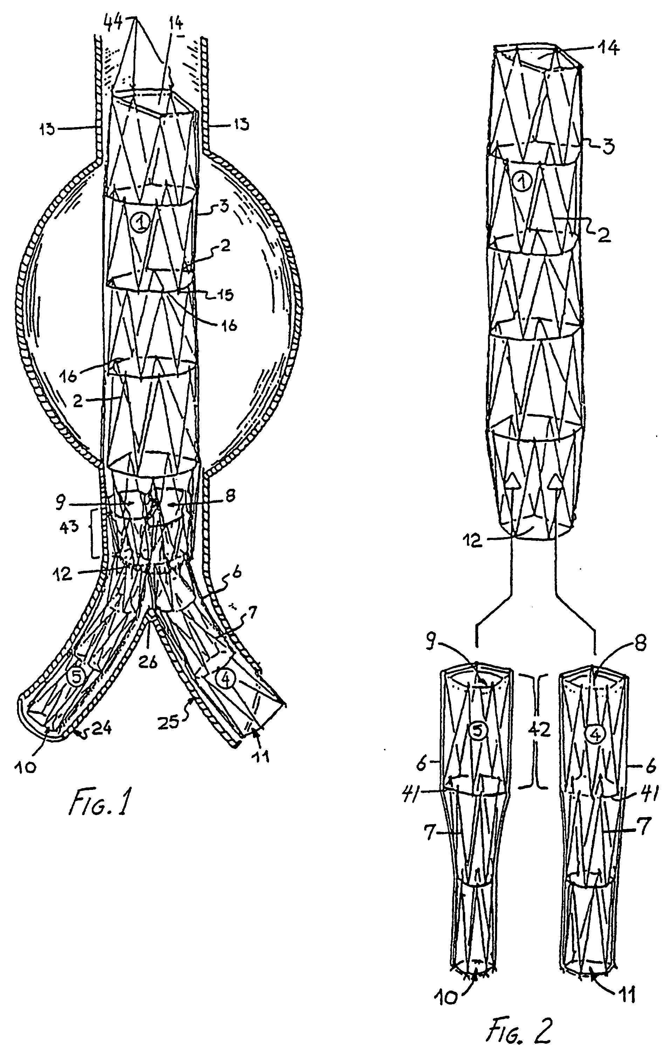

FIG. 1 is a schematic representation of the stent graft after its placement in the aneurysm of the abdominal aorta according to the present technique.
FIG. 2 is a schematic representation of the three parts of the central graft (main cylinder) and the two limbs (peripheral cylinders) from which are composed the stent graft according to the presented technique. The two limbs (peripheral cylinders) enter with their central region (of greater diameter) at the peripheral or distal region of the central cylinder and by their expansion create a leakproof branching of the central graft.

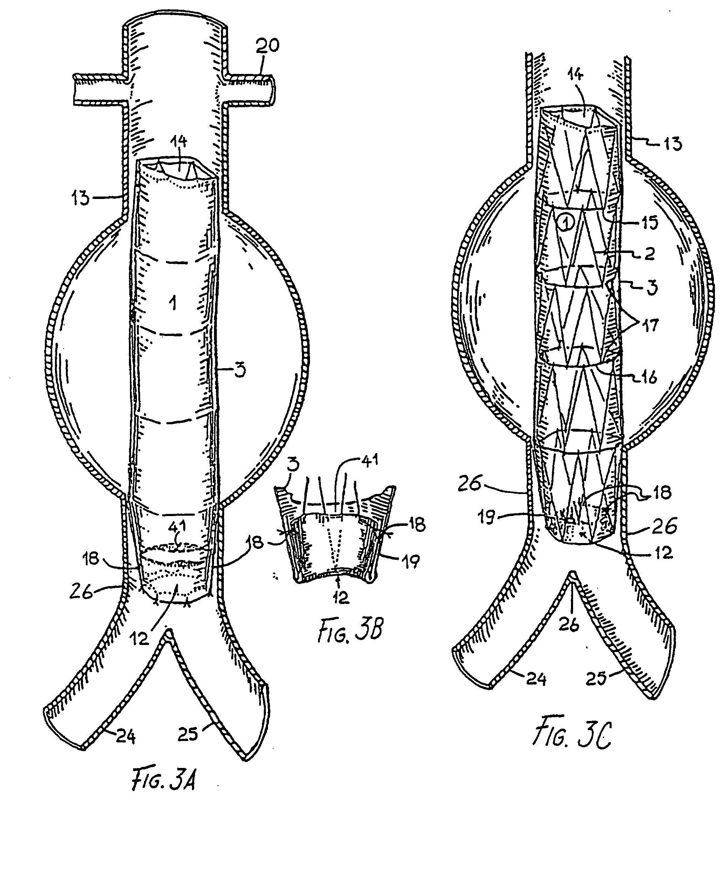

FIG. 3 is a representation of the analytical magnification of the central graft (main cylinder) which is placed in the abdominal aorta in the region between the out-branching of the kidney or renal arteries and the aortic bifurcation after its positioning and its expansion. At the peripheral end can be discerned the refolding and attachment of the thin-walled external covering inside the main cylinder, to reinforce it and to make the branching of the main cylinder more leakage proof after the placement of the two peripheral cylinders.

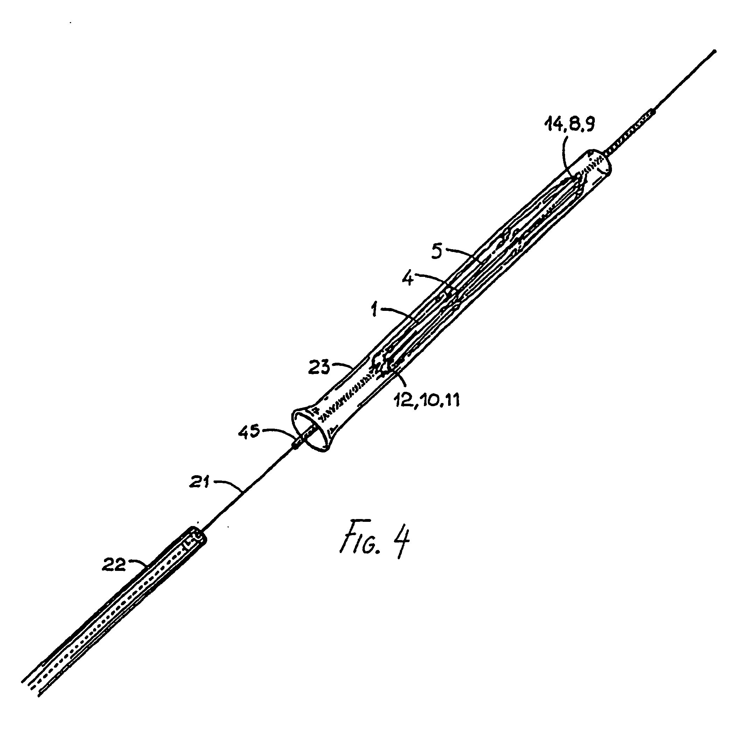

FIG. 4 is a magnified representation of the cylinders in their compressed form inside the storage tubules which are small in diameter and equal to the diameter of the placement tubules inside the arteries as well as the propulsion device for the introduction and progression of the graft by means of a guide wire.

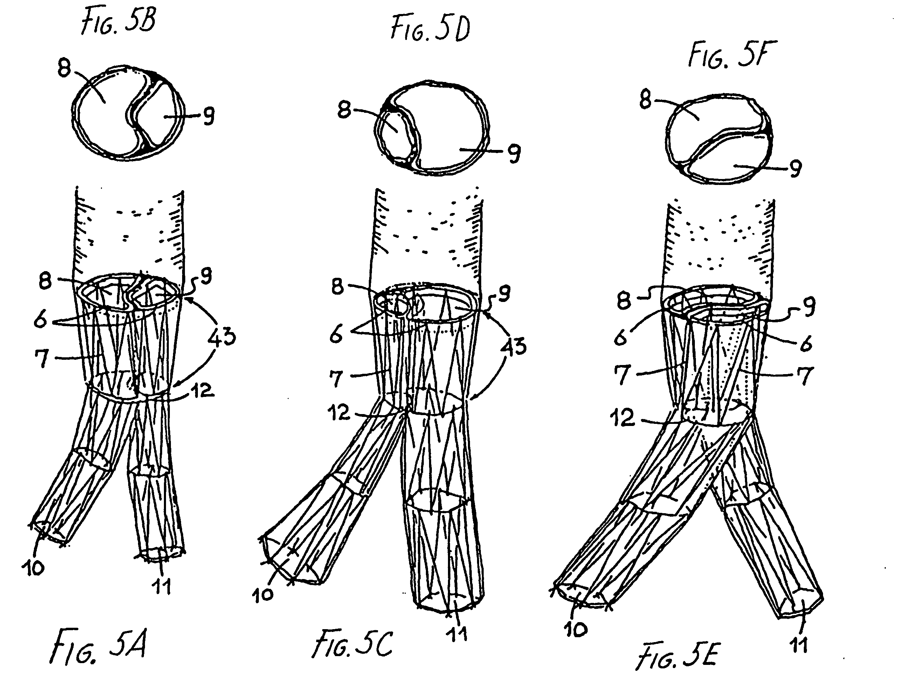

FIG. 5 is a three-dimensional schematic representation of the overlapping regions of the cylinder and of the two peripheral cylinders (limbs) after the expansion of the limbs inside the main cylinder and their leakage-proof application, not only amongst the limbs, but also with the central cylinder due to their self-expanding character. At cross-section, one can clearly see the variety of shapes that the central orifices of the limbs can take when restricted at their external surface by the internal surface of the central cylinders at their overlapping parts.

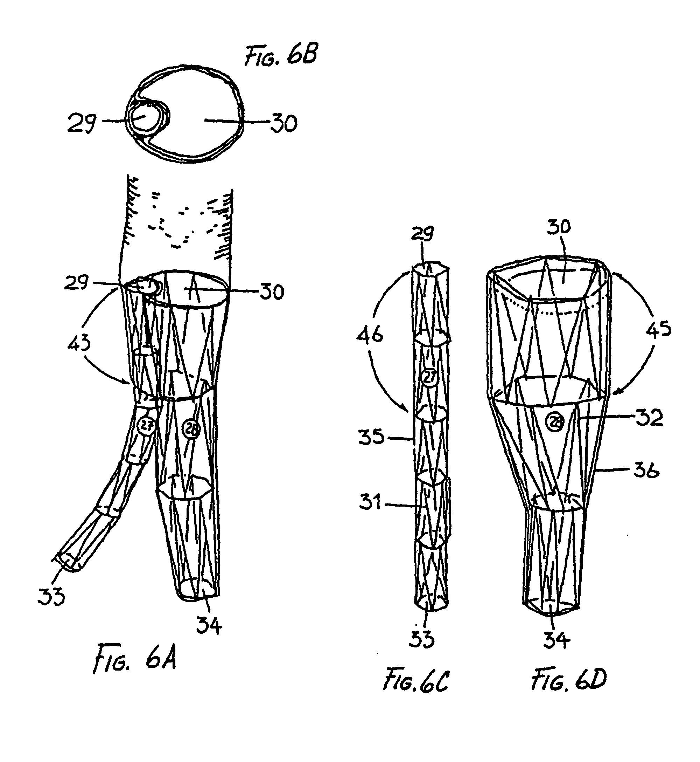

FIG. 6 is a schematic representation of an alternative manner of construction of the limbs of the stent graft where the diameter of the central orifice at one of the limbs at the length of > 2 cm (covered by the central cylindrical part) is equal to the diameter of the peripheral orifice of the main cylinder while the diameter of the other limb is smaller at the center of the orifice and part as well as at the cross-section of the central orifices after the expansion of the two limbs inside the central tube. If they were allowed to expand naturally, the cross-sectional areas of the combined limbs would be twice that of the distal, orifice or the central tube.

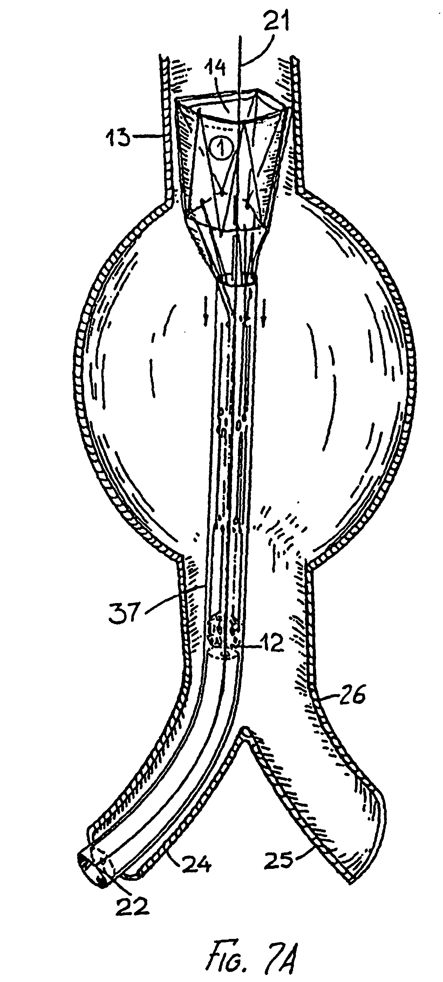

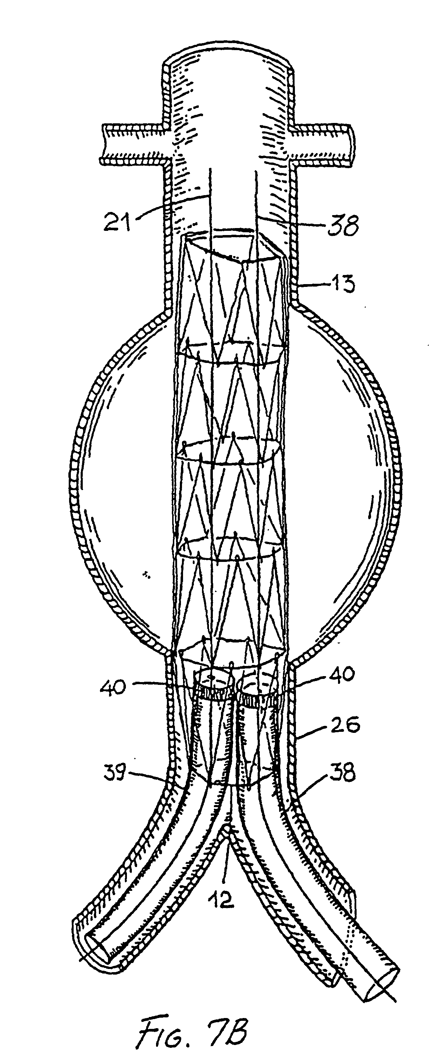

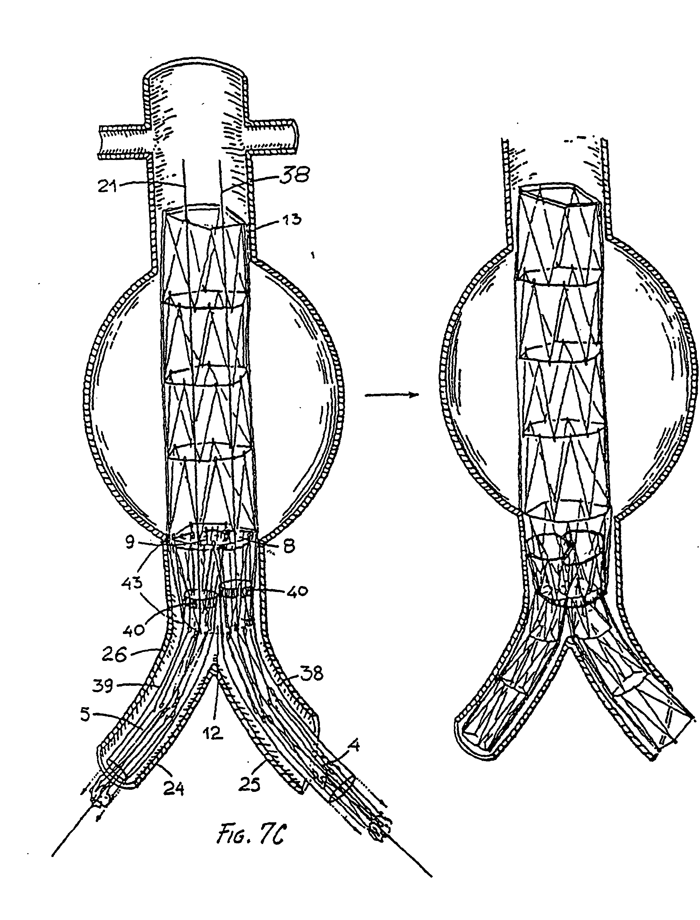

FIG. 7a is a schematic representation of the method of placement of the central graft (main cylinder). The central graft is found compressed inside the placement tubule where it is advanced since the guide wire passes through the graft and is brought to the point of placement with the help of the propulsion device and by the guide wire. The central orifice of the placement tubule has already been advanced more central to the aneurysm and when the graft reaches the position of placement, it is allowed to expand by means of maintaining the propulsion device stable and by means of the peripheral retraction of the placement tubule toward the propulsion device in such a manner so that during its expansion the graft will maintain its position unchanged.
FIG. 7b is a schematic representation after the placement of the central graft a second guide wire is advanced from the other iliac artery and by the peripheral orifice of the main cylinder. On the guide wires of both sides, the placement tubules are advanced by an X-ray shadowing ring at their central orifice, through the iliac arteries and through the peripheral orifice of the main cylinder inside the peripheral part of the main cylinder.
FIG. 7c is a schematic representation of the advancement of the two limbs inside the guide introduction tubules using the same technique as that for the placement of the central graft, and of their equal in height positioning by means of retraction of the introduction tubules when these are forwarded at an upright position which is at the same transverse level as the two limbs. The complete release of the limbs (peripheral cylinders) along with the simultaneous or the nonsimultaneous withdrawal of the introduction tubules of both sides results in the fact that the expanded parts of the limbs will come in complete contact with the peripheral nonexpanded healthy region of the corresponding iliac blood vessel at which the flow of blood is directed.

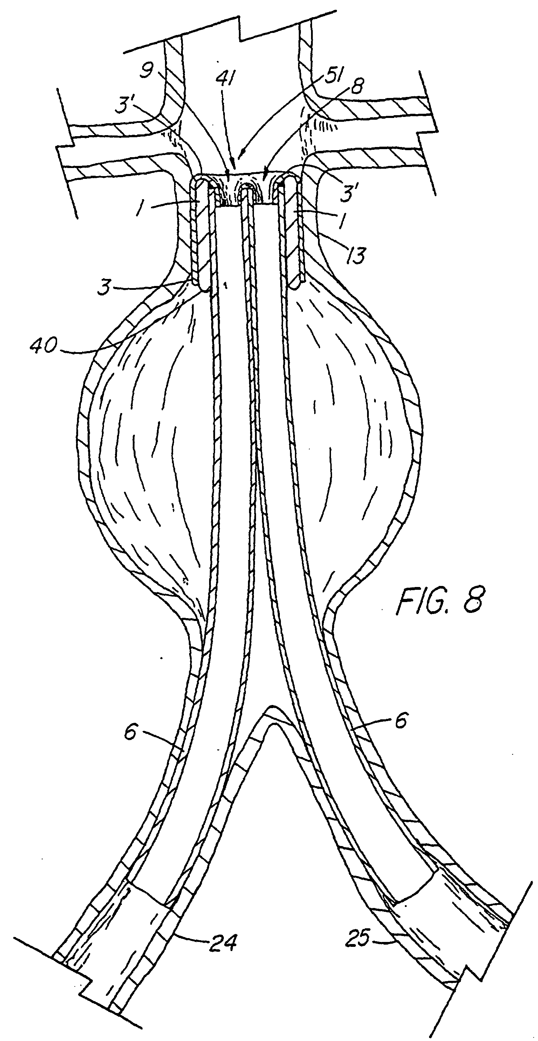

FIG. 8 illustrates an alternative arrangement in which the main graft is substantially contained within the region of the aorta containing the renal arteries. The distal end of the main graft is constructed in a manner similar to that in the other embodiments. The two limbs extend from the iliac arteries into the distal end of the main graft and are sealed in a similar manner.

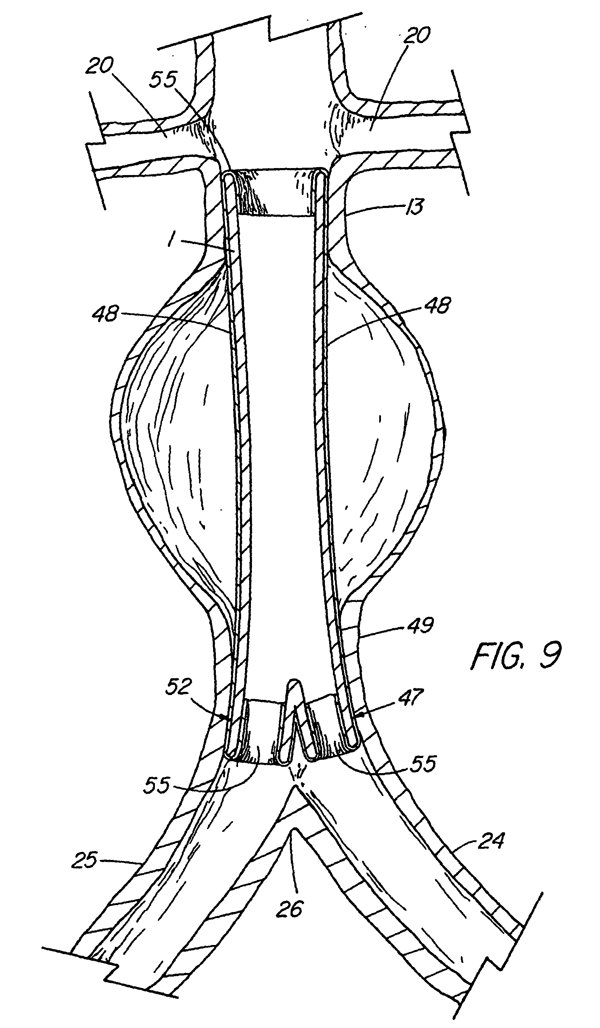

FIG. 9 illustrates another preferred alternative embodiment of the arrangement in which the bifurcated section thereof includes first and second or a pair of orifices in the form of short tubular receiving sections for receiving a respective iliac artery graft and which are to be sealed therewith.

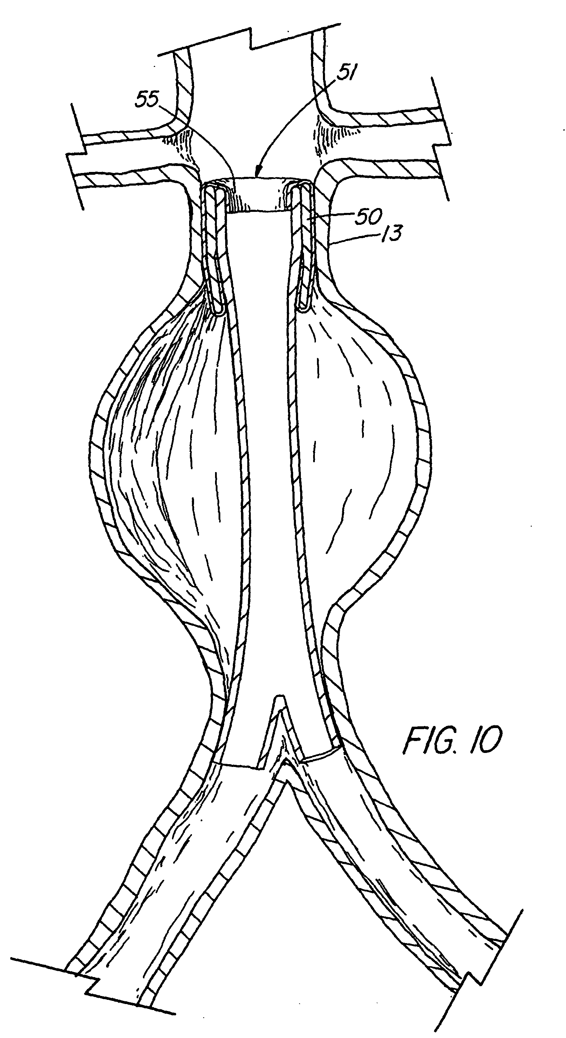

FIG. 10 illustrates the invention in which an additional engagement and sealing graft is provided at the proximal end thereof for seating the stent graft in the part of the aorta adjacent the renal arteries.

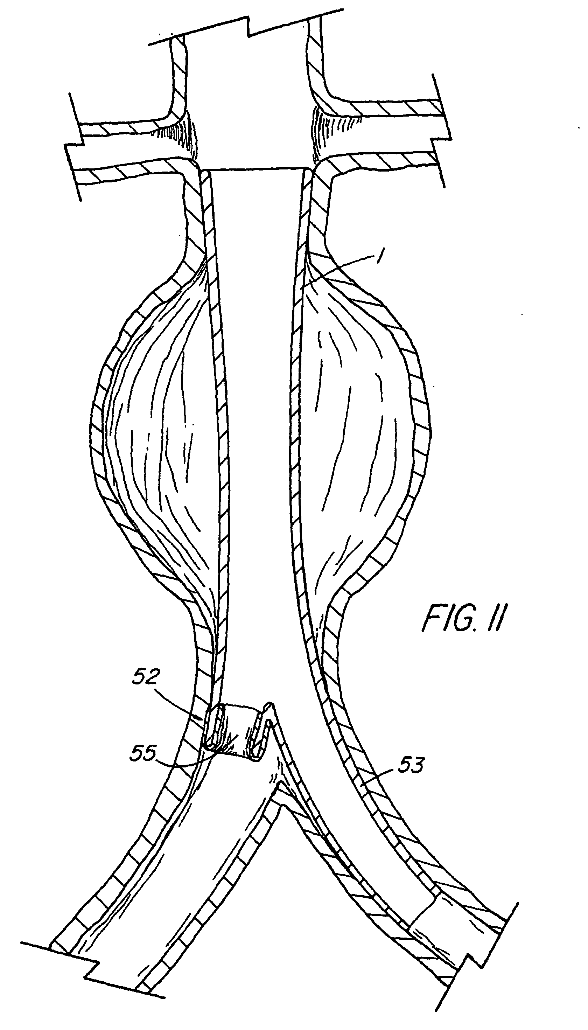

FIG. 11 illustrates yet another alternative embodiment of the arrangment similar to that of FIG. 9 in which a prefabricated iliac graft of the correct length and diameter has been affixed tothe main graft.

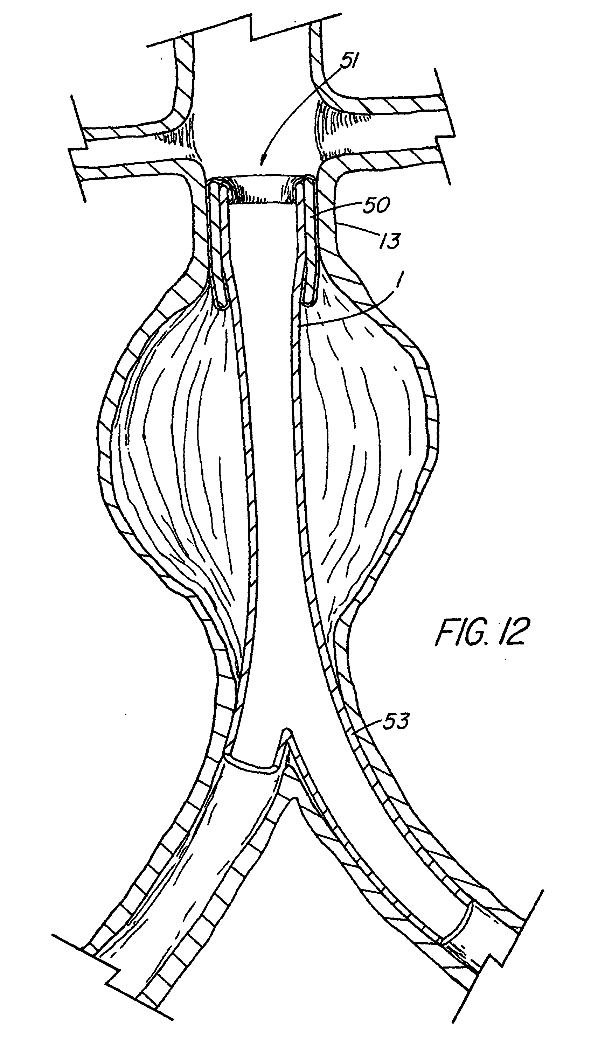

FIG. 12 illustrates still another alternative embodiment of the arrangment of FIG. 11 in which an additional engagement and sealing graft is positioned at the proximal end of the stent graft and the iliac graft has been affixed to the main graft.

### Detailed Description

In general, this device presents a stent graft for the therapy of abdominal aortic aneurysms without the need for the presence of healthy peripheral aortic walls (26), whereof the placement of one and only one graft tube would be possible. The need to avoid the pathological wall by airtightness in the nonexpanded part of the region of the iliac is accommodated by the branching of the central graft (main cylinder) at two peripheral tubes so that the blood can be driven toward both iliac arteries (24, 25) from the central aortic graft. Furthermore, the described graft must have the ability to be compressed to a small starting-off diameter (FIG. 4), such that the advancement and placement in its compressed form is made possible by means of the small diameter tubes (37, 38) inside the vascular lumen from distant regions (from the femoral artery to the abdominal aorta) where released it can regain its original large diameter. This is accomplished in such a way so that it is able to achieve its leakproof perimetric contact with the internal surface of the healthy vascular lumen central and peripheral to an aneurysm. Till today, there have been proposed and used various devices for the accomplishment of the above goals. However, these are often complicated and difficult in their usage with the subsequent appearance of complications during and after the operation. To use these devices, it is necessary for those performing the operation to acquire lengthy experience and to have ample abilities. One aim of the device, which will be described as well as its technical positioning, is the simplification of the placement procedure, the minimization of the immediate and future complications and the enhancement of the percentage of successful clinical results with the goal of the possibility of a wide usage of the method of endovascular therapy of aneurysms for those patients where the placement of a stent-graft is necessary.

The herein presented stent-graft is a graft which is comprised of a central "main" cylinder or tube (1) of which the proximal end section comes into contact with the internal surface of the healthy arterial wall of the aorta at a level more central or proximal to the distended part of the aorta (13) and below the renals while the periphery or distal end of the tube sits upon the aortic bifurcation (26). The diameter or cross-sectional area of the central or proximal orifice (14) of the main cylinder (1) is equal to or larger than the diameter of the healthy part of the aorta (13) on which the cylinder will be placed. The diameter or cross-sectional area of the peripheral or distal orifice (12) is constant and independent of whatever aortic diameter at the level of its bifurcation (26) where the cylinder will be placed. The length of the main cylinder (1) is determined by whichever length amongst the central point of contact in the aorta (13) and its bifurcation (26).

The main cylinder (1) comprises a stent or skeleton (stent) which is preferably cylindrical and/or metallic with a length that is substantially equal to the distance between the kidney or renal arteries and the aortic bifurcation. This skeleton has as starting-off predetermined diameter α and a compressed diameter β and consists of successive and serially connected cylindrical Z stents of variable length and with a Z configuration made out of biocompatible metal with memory such as stainless steel wire or nitinol (a nickel titanium alloy with thermal memory). These Z stents are preferrably of the Gianturco type and are described in U.S. Patent No. 4,580,568. The connection of these parts of the skeleton can be accomplished either by sutures (16) which pass through the orifices of the last of the endcrests (15) of each Z stent or by metallic joints (solderings) in such a manner so as to allow a certain amount of flexibility amongst the various Z stents of the skeleton whilst the length of the skeleton has as minimal as possible changes between its compressed and its starting-off diameter. The present plan of the skeleton is described extensively and includes of the skeleton (2) of the main cylinder (1) which is described in the figures as well as other parts hereof. However, other self-expanding skeletons with similar properties and characteristics which will possibly save the basic idea of the creation of the bifurcation of the herein presented stem-graft are also contemplated. The stent can at its proximal end, have a plurality of spikes, barbs or hooks which can, upon expansion and/or rotation penetrate part (13) for sealing and securement thereto.

The metallic skeleton or stent of the main cylinder on its outer surface is covered by a tube (3) to form a graft which has a central (14) orifice, and a peripheral or distal (12) orifice. The wall is preferably thin-walled and of polytetrafluoroethylene (PTFE), Dacron™, polyurethane or another type of biocompatible plastic. Alternatively, the inner and outer surfaces of the metallic skeleton (2) can be covered by cylindrical tubes (3), or it can be covered on its inner surface only by the cylindrical tube. This tube has a starting-off or proximal diameter which is equal to that of the metallic skeleton and it has a central and peripheral distal orifice and a main body. The central orifice (14) has a diameter which is preferably substantially equal to or greater than that of the healthy part of the aorta at the point of its contact (13) with the main cylinder more central (proximal) to the aneurysm. The tube(s) of the graft is refolded or is not refolded at the central (proximal) orifice (14) of the metallic skeleton (2) and is attached upon the central orifice of the skeleton by a series of connective sutures (44) at the end-crests of the central (proximal) end of the (metallic) skeleton or stent. An outer covering can cover an outer enlargement at the outer proximal end of cylinder (1) to firmly engage part (13) and then a flap can extend inwardly into orifice (14) to improve the seal. The peripheral orifice of the graft has a diameter of 20 - 25 mm and is refolded (18) at a length of 0.5 -1.0 cm at the internal side of the peripheral or distal orifice (12) of the metallic skeleton where it is internally attached by single sutures at two or at three different points of the metallic skeleton (2). Thus, the flow of blood after the placement of the main cylinder is accomplished inside the peripheral refolding of the graft of the main cylinder at the peripheral (distal) end and creates two or three pockets (petals) (41) minimizing the surface of the peripheral orifice (12) of the main cylinder. Additional attachment points can be used, if desired, to establish additional petals. The main cylinder (1) has a starting-off diameter, which is of the thin-walled covering cylinder (3) around the center (14) and periphery (12) of the orifice end around its body. The metallic skeleton (2) expands the cylinder to this diameter and to a compressed diameter much smaller than that of the original diameter in such a manner so that it can be compressed inside the storage tubule (23) which has a small diameter (FIG. 4) equal to that of the placement tubule (37) which is used for the advancement of the graft inside the blood vessels (FIG. 7a). During the compression of the main cylinder (1) inside the storage tubule (23) it has at its center a catheter (39) which is used for the Insertion of the guide wire (21) (which after this it is removed) through the compressed inside of the storage tubule and main cylinder during the positioning process. The progression of the main cylinder to the placement position is accomplished by its propulsion by the storage tubule (23) to the placement tubule (37), which as previously mentioned, has the same diameter. This is achieved with the help of a propulsion device (22) which moves upon the guide wire (21) which goes through the center of the main cylinder and in continuation through the aneurysm.

The herein presented stent-graft is also comprised of two peripheral cylinders (limbs) (4, 5) which preferably are self-expanding stents or skeletons (7) identical to that of the main cylinder (1) but of different dimensions (a series of Z stents). These are covered at their external surface by a thin-walled cylindrical tube (6), which have an identical or a different composition from that of the main cylinder (1), but are of different dimensions. The peripheral cylinders have central (proximal) (8, 9) and peripheral (distal) (10, 11) orifices and a starting-off diameter and a compressed diameter such that it becomes possible for them to be placed inside a storage tubule (23) exactly In the same way as with the main cylinder but of a smaller diameter. They can be advanced to the corresponding placement tubules (37, 38) inside of the blood vessels. The thin-walled cylindrical tube (6) covers the entire length of the metallic skeleton (7) and is attached to the central (8, 9) and the peripheral (10, 11) end of the metallic skeleton of each peripheral cylinder. The skeleton (7) of the peripheral cylinders (4, 5) has a diameter in its expanded form equal to or greater than that of the thin-walled cylindrical tube (6) at each of its parts in such a manner so that it comes in complete contact at its external surface with the internal surface of the thin-walled cylindrical tube (6) which has a constant diameter at its expanded form and at each of its parts. The graft can be refolded to a small length < 5mm and can also not be refolded inside the central orifice (8, 9) of the skeleton of each limb and is not refolded at the peripheral orifice (10, 11 ) of the skeleton of each limb where it is attached by sutures.

The diameter or diameters of the central orifice (8, 9) of each (peripheral cylinders) limb is preferably equal to or approximately up to about 5 mm or more smaller in relation to the diameter of the peripheral orifice (12) of the main cylinder. Alternatively, each orifice (8,9) can be equal to or greater than orifice (12), or each can be significantly smaller than orifice by much more than 5 mm such as 10 or 20 mm. Experimentation of a simple nature can determine sizes of the distal cylinders relative to orifice (12) in order to achieve a sealing effect between cylinders (1, 4 and 5). The diameter of the central orifice of each limb continues at a length of 2 - 2.5 cm at the central part (42) of the graft of each limb which is the length of the first of the Z stents of the preferably self-expanding stents or skeletons (7) of each limb and the point (17) where the first stent of the preferably metallic skeleton is connected to (jointed to) the second stent as has previously been mentioned. This central part (42) of each limb is the part which enters into the distal or peripheral end (43) of the central cylinder for the creation of the bifurcation of the central cylinder as will be mentioned in continuation. The diameter of the peripheral cylinders, more peripheral to the previously mentioned part, has a length and a diameter which vary according to the length and the diameter that is necessary so that the peripheral orifice (10, 11) of each of those two cylinders (4, 5) can come in complete contact with the healthy part of the corresponding iliac blood vessel (24, 25). That is to say, that the peripheral diameter and the length of each limb can differ from each other in relation to the dimensions of the iliac blood vessels of their healthy part and of the length of the damage of each, from the bifurcation of the aorta.

Alternatively, the diameter of the central orifice of the central part of each limb can differ in size FIG. 6. Specifically, the diameter of the central orifice (30) and of the central part (46) of one of the limbs (28) which enters from the peripheral orifice (12) of the main cylinder (1) is equal to the diameter of the peripheral part and of the orifice (12) of the main cylinder, while the diameter of the central part (46) and the orifice (29) of the other limb (27) can be smaller with the aim of creating a smaller compressed limb diameter and its progression inside of a smaller in diameter placement tubule, percutaneously. In this case, the expanding ability of the metallic skeleton (31) of the smaller in diameter limb is equal to or greater than that of the metallic skeleton (32) of the limb with the greater central diameter. The length as well as the periphery of the peripheral orifice (33, 34) of each peripheral cylinder (limb) (27, 28) can vary as previously mentioned according to the dimensions of the iliac blood vessels and their condition.

As material for the thin-walled covering cylinder (6, 35, 36) of the metallic skeleton (7, 31, 32) of the peripheral cylinders (4, 5, 27, 28) one can use a cylinder made out of thin-walled polytetrafluoroethylene, Dacron™, or another type of biocompatible plastic. This thin-walled cylinder preferably has the previously mentioned constant dimensions of the peripheral cylinders (4, 5, 27, 28) at its noncompressed form as well as after its expansion by the self-expanding metallic skeleton (2) Internally. The material of the thin-walled covering cylinder (6, 35, 36) of the metallic skeleton of the peripheral cylinders can cover the cylindrical metallic skeleton at its external surface or at its external and internal surface or at its internal surface.

### Technical Placement - Creation of a Bifurcation

The herein presented stent-graft is created by the placement of three cylinders (1,4,5) (main and two peripheral) of which it is composed and which is executed in the following manner:

After the percutaneous placement of the guide wire (21) from the femoral artery and in a head on direction towards the aorta, an angiogram is performed so as to determine the height of the kidney arteries (20). At this point, as well as at the point of the aortic bifurcation, the X-ray shadowed guided position is monitored by X-rays. On the guide wire is advanced with the help of a diagnostic device, percutaneously, the introduction tubule (37) (sheath) inside of which the main cylinder will be advanced during its placement. The introduction tubule has at its central end an X-ray shadowed ring (40) and at its peripheral end it has a hemostatic valve. During its endovascular advancement, the introduction tubule (37) has inside of it a diagnostic device which makes easier its percutaneous entrance into the artery. The introduction tubule is advanced inside the aorta so far in as necessary so that the X-ray shadowed ring at its central end will be found at a more central level than that of the out-branching of the kidney arteries (20). In continuation, the diagnostic device is removed from inside of the introduction tubule (37). And on the guide wire (21) through the hemostatic valve, the storage tubule (23) is advanced which has a diameter equal to that of the introduction tubule which carries inside its lumen the compressed main cylinder (1) (of the stent graft) that has a length equal to that of the distance amongst the point of the out-branching of the kidney arteries (20) and the aortic bifurcation (26) and a diameter of the central orifice (14) (after its expansion) which is equal to or greater than that of the aorta at the height of the central neck (13) directly underneath the kidney tubules.

The main cylinder (1) is advanced from the storage tubule (23) to the introduction tubule (37) and through this to the placement point with the help of a propulsion column (propulsion device) (22), which passes through the center of the compressed main cylinder. When the main cylinder (1) which stents an X-ray shadow in its entire length (metallic skeleton) is advanced inside the introduction tubule (37) between the guide points that have been placed at the out-branching of the kidney arteries (20) and the aortic bifurcation (26), the propulsion column (22) is maintained in a stable condition by its central end which restrains the peripheral end (12) of the main cylinder at the height of the aortic bifurcation. The introduction tubule (37) is retracted over the column (22) in a centrifugal direction in such a manner so that the main cylinder (1) is progressively released from the introduction tubule at its entire length and will expand (FIG. 7a). When it comes into contact with the internal surface (13) of the aorta directly beneath the kidney arteries (20) it will become more rounder whilst the peripheral orifice sits upon the aortic bifurcation (26). In this way, the dislocation of the main cylinder becomes impossible due to the constant length of the metallic skeleton (2) of the main cylinder which is supported by the aortic bifurcation.

In continuation and after the de novo progression of the column inside the introduction tubule (37), it is advanced on the guide wire (21) through the peripheral orifice (12) inside of the main cylinder (12) so that the X-ray shadowing ring (40) will be found 2 - 2.5 cm more central to the peripheral orifice (12) of the main cylinder.

In the same way, one of the two peripheral cylinders (5) is advanced inside of the introduction tubule through the hemostatic valve. In this manner, its central end (orifice) (9) will reach the height of the X-ray shadowing ring (40) of the introduction tubule.

In continuation, percutaneous advancement of the guide wire (38) is achieved from the femoral artery of the other side after its percutaneous injection. The guide wire is centripetally directed with the help of a guide catheter through the iliac artery (25) and through the peripheral orifice of the main cylinder which sits upon the main cylinder which sits upon the aortic bifurcation inside the lumen of the main cylinder. Advanced on the guide wire follows the second introduction tubule (38) with a diagnostic device inside of it and an X-ray shadowing ring (40) at its central orifice. The second introduction tubule (38) is centripetally advanced through the peripheral orifice (12) of the main cylinder and up to the point where the X-ray shadowing ring at its central orifice is found at the same height (2 - 2.5 cm from the peripheral orifice inside the main cylinder) with the X-ray shadowing ring of the main orifice of the introduction tubule (37) of the femur of the other side (FIG. 7b) inside of which is already found the peripheral cylinder of the limb (5) of the other side at its compressed form. Inside the second introduction tubule end with the technique which was previously mentioned, the second peripheral cylinder (limb) (4) is advanced till the point where its central compressed end is at an equal height as that of the X-ray shadowing ring (40) of the introduction tubule (38) inside of which it is advanced as well as with the central end of the compressed peripheral cylinder (5) of the other side.

After they are X-ray monitored, the two compressed peripheral cylinders (limbs) inside of the introduction tubules have a position of equal height, both with their central end, 2-2.5 cm more central and inside of the peripheral orifice of the main cylinder; that is to say, at the point of the union (41) (joint) of the first with the second Z element of their metallic skeleton which has a corresponding length, the introduction tubules are withdrawn simultaneously or nonsimultaneous in a centrifugal or distal direction and the peripheral cylinders are expanded according to the technique which was mentioned previously for the main cylinder.

After the expansion, the two peripheral cylinders, these having a self-expanding skeleton, preferably each with an equal strength of expansion at the end covered by their main cylinder part extended, are compressed due to the greater total diameter of both in relation to the diameter of the peripheral part of the central cylinder by the main cylinder. In this way, they come into leak-proof contact with the internal surface of the wall of the central cylinder, but also between them but however, maintaining the diameter of both central orifices (8, 9) equal to the diameter of the peripheral part (43) of the main cylinder (1). The shape of the central orifice of the two peripheral cylinders can vary, without these, however, coinciding completely due to the equivalent expansive ability of their metallic skeletons (FIG. 5).

Furthermore, the reversal of the external cover (19) of the main cylinder at the peripheral orifice (12) creates an additional valve mechanism at this level which hinders the escape of blood from the microchasms which may occur during the contact amongst the two peripheral cylinders, but also with the internal surface of the peripheral part (43) of the main cylinder.

In this manner, a blood leak-proof (without the escape of blood) bifurcation of the main cylinder (1) is created at two peripheral cylinders (5, 4) with an entrance orifice (9, 8) of variable shape and area.

The peripheral part of each peripheral cylinder (limb) has a length and a diameter of the peripheral orifice which is analogous to those of the corresponding iliac artery (24, 25), so that after its expansion, it will come in complete contact with the internal surface of the healthy part of the wall of the iliac artery.

After the placement of the "stent graft", according to the manner which was previously mentioned, the direction of the flow of blood is achieved inside of the "stent" graft from the height of the kidney arteries through the orifice of the two limbs and more peripheral to these inside of the iliac arteries with the simultaneous exclusion of the systemic arterial pressure end the blood circulation of the pathologically distended wall of the aneurysm of the aorta which also includes the aortic bifurcation.

In the FIG. 8 embodiment, the graft arrangement comprises a main graft 50 which is formed in a manner similar to graft 1 of the other embodiments. It comprises an inner stent and an outer covering 3, the letter extending upward to form a flap 3' which when the assembly is operational, folds into the graft limbs to assist the sealing process.

The main graft 50 is introduced into the part 13 of the aorta with the proximal end 51 of the graft adjacent to the renal arteries and with the distal end 40 of the graft adjacent to the distal end of the good tissue 13. The graft is expandable to form a force fit within part 13. If desired the graft 50 can be provided with spikes, barbs and/or hooks, which upon expansion of graft 50, rotate and embed themselves into part 13 in a known manner. The graft 50 has an internal cross-sectional area at least at end 54, much less than the sum of the external cross-sectional areas of the limb members 6. The latter are introduced via their respective iliac arteries in a tube such as 37 and allowed to expand and for the seals with the main cylinder 50 and using the flap 3' of the cover extending beyond the provisional end of the cylinder 50. The latter is quite stable since it is supported by the firm or undiseased part 13 of the aorta and it's position is quite fixed. The round flap can have 2 slits at appropriate positions to facilitate entry into the two respective tubes.

In FIG. 9, a main graft 1 extends from the part 13 of the aorta containing the renal arteries 20 to its distal end in the region of the bifurcation 28 of the iliac arteries 24, 25 with the lower end 49 of the aorta. The said distal end contains a bifurcated section with two orifices in the form of short tubular receiving sections 47, 52 each for receiving a respective iliac artery graft, and to be sealed therewith.

Each graft contains an expandable stent which is preferably self expandable, but which can be expandable by other means, such as an inflatable balloon. Each stent is preferably made of stainless steel or nitinol which are self expandable, and has at least one coating or cover 48 over and/or in the stent. The outer cover 48 on the receiving sections 47, 52 extends past the distal ends of these sections and extends in a proximal direction inside these sections. When each iliac graft is inserted into a respective section, the proximal end of the bend over cover enters the proximal end of the respective iliac graft and thereby forms an additive seal.

The cross-sectional area of the proximal end of each iliac graft, when expanded is sufficiently greater than the cross-sectional area of the associated receiving section, so that when the iliac graft is in position in the section and expanded, it exerts an outward force against the sections to form a seal therewith. The proximal end of the iliac graft is of a shape similar to that of the respective receiving section and that enables an efficient seal to be achieved. The fold-over onto cover provides an additional seal.

Each receiving section 47, 52 is prefabricated with a special shape and with reinforcement to cope with the outward force exerted by the proximal end of the respective iliac graft. One preferred form of reinforcement is achieved by a folding over 55 of an inner cover, and the section preferably has an inwardly shaped orifice at the distal end of the section to provide an extra clamping effect with the iliac graft.

The proximal end of the main graft can have a bent over section 55 of the outer cover to provide an additional seal at part 13. The stent at the proximal end of the main graft can have hooks or barbs to clamp onto the part 13, such barbs rotating into an outward engaging direction upon expansion of the stent in a known manner.

An inventive engagement and sealing graft arrangement is shown in FIG. 10 in which an additional relatively short graft 50 is provided by percutaneous iliac insertion to seal in the part 13. Graft 50 can be longer or shorter than the part 13 and is provided to add extra strength to the seal between the proximal end 51 of the main graft and the part 13. The graft 50 has a stent and preferably an extended cover 48 (not shown) to provide the extra sealing effect, and barbs for adhesion to the part 13. The distal end of graft 50 has a reduced cross-sectional area, as shown, and has folded over covers to provide extra strength. Clearly, other forms of reinforcement of the graft 50 can be provided. In the embodiments shown in FIGs. 9 and 10, the iliac grafts can be percutaneously introduced into the expanded orifice sections when the iliac grafts are compressed as described previously. Upon expansion, an efficient-seal with fold over cover 48 is achieved and the procedure is relatively simple compared to prior known procedures.

FIG. 11 shows an arrangement similar to FIG. 9 except that a prefabricated iliac graft 53 of correct selected length and diameter is fixed to the main graft. The prefabricated graft 53 replaces the receiving section 47, and is automatically sealed to the iliac artery at an appropriated distal position in that artery. This embodiment has the advantage of reducing the procedure time. The main graft is constructed and Installed in the same manner as the graft 1 shown in FIG. 9. A cover flap 55 is preferably provided at the proximal end and at the receiving section 52.

The embodiment shown in FIG. 12 is the same as that shown in FIG. 11 except that the additional graft 50 of the FIG. 10 arrangement has been included. Parts 50, 51 and 13 operate in the same way as previously described.

In the above described embodiments, self expanding stents have been employed. If that is not desirable for any reason, then non sell expanding stents can be employed, but that would necessitate the use of expansion means sucn as balloons could be used, such as those employed in angioplasty procedures.

## Claims

1. An endovascular stent graft arrangement (1, 4, 5) comprising an expandable main stent graft (1) to be endovascularly introduced into the aorta and having a first end (14) to be located in and, when expanded, to be supported by a part of the aorta adjacent to the renal arteries, the main stent graft (1) also having at least one second end (12), which when expanded serves to receive a first end (8, 9) of at least one expandable iliac artery stent graft (4, 5), wherein the main stent graft (1) and the said at least one iliac artery stent graft each comprise at least one expandable stent and at least one cover over and/or in the stent, and wherein the cross-sectional area of the at least one second end (12) of the main stent graft (1), when expanded, is sufficiently less than the cross-sectional area of the at least one peripheral iliac artery graft when expanded in order to form a seal between the grafts, **characterised by** an additional stent graft (50) having a proximal orifice region and a distal orifice region, for insertion and expansion into the said part of the aorta, and for surrounding the first end of the main stent graft (1), and **characterised in that** the said additional stent graft (50) has a cover over and/or in the stent that extends over the proximal end of the additional stent graft (50), and **in that** the additional stent graft (50) has a diameter of its distal end which is less than the diameter of its proximal end, so as to reinforce the distal end of the additional stent graft (50) and to provide support for the first end of the main stent graft (1).

2. The stent graft arrangement (1, 4, 5) according to claim 1, wherein the at least one second end (12) of the main stent graft (1) has a part (53) which is extended to form an iliac artery stent graft, and another part (52) which has a distal opening or orifice which'has a short inclined extension subtended in a distal direction so as to enable an expandable iliac artery stent graft (4, 5) to be located therein when the short extension has been expanded, such expandable iliac artery stent graft (4, 5) having a first end (8, 9) which when expanded will form a seal with the short extension.

3. The stent graft arrangement (1, 4, 5) according to claim 1, wherein the at least one second end (12) of the main stent graft (1) has first and second short extensions (47, 52), one extension (47) having said at least one distal orifice and the other extension (52) having another distal orifice for the receipt of respective expandable iliac artery stent graft (4, 5), each of which having a stent expandable to a diameter such that each expandable iliac artery stent graft (4, 5) has a cross sectional area sufficiently greater than the cross-sectional area(s) of the said distal orifices when the short extensions have been expanded, in order to form effective seals.

4. The stent graft arrangement (1, 4, 5) according to any one of the preceding claims, wherein the stent(s) of the said additional stent graft (50) is supplied with spikes, barbs, or hooks to facilitate securement of the aorta.

5. The stent graft arrangement (1, 4, 5) according to any one of the preceding claims, wherein the reinforcement at the second end of the additional stent graft (50) is provided by folds (50) in an internally folded cover (48) in order to provide an increased thickness of cover material.

6. The stent graft arrangement (1, 4, 5) according to any one preceding claims wherein the external cover (48) has a proximal extension on the outer member of each seal whereby said extension is designed folded over the proximal end of the outer member and entering the proximal end of the internal member of each seal, such folded over cover providing an extra sealing facility.

## Patentansprüche

1. Endovaskuläre Stentimplantat-Anordnung (1, 4, 5) mit einem expandierbaren Haupt-Stentimplantat (1), das endovaskulär in die Aorta eingeführt werden soll und ein erstes Ende (14) aufweist, das in einem Teil der Aorta neben den Nierenarterien angeordnet werden soll und nach der Expansion darin gestützt wird, wobei das Haupt-Stentimplantat (1) ferner zumindest ein zweites Ende (12) aufweist, das nach der Expansion zur Aufnahme des ersten Endes (8, 9) mindestens eines expandierbaren Iliaka-Stentimplantats (4, 5) dient, worin das Haupt-Stentimplantat (1) und das mindestens eine Iliaka-Stentimplantat jeweils mindestens einen expandierbaren Stent und mindestens eine Abdeckung über dem und/oder im Stent umfassen und worin der Querschnittsbereich des mindestens einen zweiten Endes (12) des Haupt-Stentimplantats (1) nach der Expansion so viel kleiner ist als der Querschnittsbereich des mindestens einen peripheren Iliaka-Stentimplantats nach der Expansion, dass er eine Dichtung zwischen den Implantaten bildet, **gekennzeichnet durch** ein zusätzliches Stentimplantat (50) mit einer proximalen Öffnungsregion und einer distalen Öffnungsregion zum Einführen und Expandieren in jenem Teil der Aorta und zum Umgeben des ersten Endes des Haupt-Stentimplantats (1) und **dadurch** gekennzeichnet, dass das zusätzliche Stentimplantat (50) eine Abdeckung über dem und/oder im Stent aufweist, die sich über das proximale Ende des zusätzlichen Stentimplantats (50) erstreckt und dass das zusätzliche Stentimplantat (50) einen Durchmesser an seinem distalen Ende aufweist, der kleiner ist als der Durchmesser seines proximalen Endes, um das distale Ende des zusätzlichen Stentimplantats (50) zu verstärken und eine Stütze für das erste Ende des Haupt-Stentimplantats (1) zu bilden.

2. Stentimplantat-Anordnung (1, 4, 5) nach Anspruch 1, worin das mindestens eine zweite Ende (12) des Haupt-Stentimplantats (1) einen Teil (53) aufweist, der verlängert ist, um ein Iliaka-Stentimplantat zu formen, sowie einen anderen Teil (52), der eine distale Öffnung oder Aussparung aufweist, die eine in einer distalen Richtung begrenzte kurze geneigte Verlängerung aufweist, damit ein expandierbares Iliaka-Stentimplantat (4, 5) darin angeordnet werden kann, wenn die kurze Verlängerung expandiert ist, wobei dieses expandierbare Iliaka-Stentimplantat (4, 5) ein erstes Ende (8, 9) aufweist, das nach der Expansion eine Dichtung mit der kurzen Verlängerung bildet.

3. Stentimplantat-Anordnung (1, 4, 5) nach Anspruch 1, worin das mindestens eine zweite Ende (12) des Haupt-Stentimplantats (1) eine erste und zweite kurze Verlängerung (47, 52) aufweist, wobei eine Verlängerung (47) die mindestens eine distale Öffnung aufweist und die andere Verlängerung (52) eine andere distale Öffnung zur Aufnahme des jeweiligen expandierbaren Iliaka-Stentimplantats (4, 5) aufweist, jeweils mit einem Stent, der auf einen solchen Durchmesser expandiert werden kann, dass jedes expandierbare Iliaka-Stentimplantat (4, 5) einen Querschnittsbereich aufweist, der so viel größer ist als der Querschnittsbereich bzw. die Querschnittsbereiche der distalen Öffnungen, wenn die kurzen Verlängerungen expandiert sind, dass er wirksame Dichtungen bildet.

4. Stentimplantat-Anordnung (1, 4, 5) nach einem der vorhergehenden Ansprüche, worin der Stent bzw. die Stents des zusätzlichen Stentimplantats (50) mit Dornen, Widerhaken oder Haken versehen sind, um die Fixierung der Aorta zu erleichtern.

5. Stentimplantat-Anordnung (1, 4, 5) nach einem der vorhergehenden Ansprüche, worin die Verstärkung am zweiten Ende des zusätzlichen Stentimplantats (50) durch Falten (50) in einer innen gefalteten Abdeckung (48), um dem Abdeckmaterial eine größere Dicke zu verleihen, gebildet wird.

6. Stentimplantat-Anordnung (1, 4, 5) nach einem der vorhergehenden Ansprüche, worin die äußere Abdeckung (48) eine proximale Verlängerung am äußeren Element jeder Dichtung aufweist, wobei die Verlängerung über das proximale Ende des äußeren Elements gefaltet ist und in das proximale Ende des inneren Elements jeder Dichtung eindringt, wobei diese gefaltete Abdeckung ein zusätzliches Mittel zur Abdichtung darstellt.

## Revendications

1. Dispositif constitué d'une endoprothèse vasculaire (1, 4, 5) comprenant une endoprothèse principale dilatable (1) destinée à être introduite par voie endovasculaire dans l'aorte et comportant une première extrémité (14) destinée à être positionnée dans et, quand elle est dilatée, à être supportée par, une partie de l'aorte adjacente aux artères rénales, l'endoprothèse principale (1) comportant aussi au moins une deuxième extrémité (12) qui, quand elle est dilatée, sert à recevoir une première extrémité (8, 9) d'au moins une endoprothèse dilatable d'artère iliaque (4, 5), dans lequel l'endo-prothèse principale (1) et ladite endoprothèse d'artère iliaque au moins comprennent chacune au moins un stent dilatable et au moins une enveloppe par-dessus et/ou dans le stent, et dans lequel la superficie de la section de ladite deuxième extrémité au moins (12) de l'endoprothèse principale (1), quand elle est dilatée, est suffisamment inférieure à la superficie de la section de ladite endoprothèse d'artère iliaque périphérique au moins quand elle est dilatée de façon à former un joint d'étanchéité entre les endoprothèses, **caractérisé par** une endoprothèse additionnelle (50) comportant une zone constituée d'un orifice proximal et une zone constituée d'un orifice distal pour l'insertion et la dilatation dans ladite partie de l'aorte, et pour entourer la première extrémité de l'endoprothèse principale (1), et **caractérisé en ce que** ladite endoprothèse additionnelle (50) comporte une enveloppe par-dessus et/ou dans le stent qui se prolonge par-dessus l'extrémité proximale de l'endoprothèse additionnelle (50), et **en ce que** l'endoprothèse additionnelle (50) a un diamètre de son extrémité distale qui est inférieur au diamètre de son extrémité proximale, de façon à renforcer l'extrémité distale de l'endoprothèse additionnelle (50) et à fournir un support pour la première extrémité de l'endoprothèse principale (1).

2. Dispositif constitué d'une endoprothèse (1, 4, 5) selon la revendication 1, dans lequel ladite extrémité au moins (12) de l'endoprothèse principale (1) comporte une partie (53) qui est étendue pour former une endoprothèse d'artère iliaque, et une autre partie (52) qui comporte une ouverture ou un orifice distal(e) qui possède une extension courte inclinée sous-tendue dans une direction distale de façon à permettre de positionner une endoprothèse dilatable d'artère iliaque (4, 5) dedans quand l'extension courte a été dilatée, cette endoprothèse dilatable d'artère iliaque (4, 5) comportant une première extrémité (8, 9) laquelle, quand elle est dilatée, forme un joint d'étanchéité avec l'extension courte.

3. Dispositif constitué d'une endoprothèse (1, 4, 5) selon la revendication 1, dans lequel ladite deuxième extrémité au moins (12) de l'endoprothèse principale (1) comporte une première et une deuxième extensions courtes (47, 52), une extension (47) comportant ledit orifice distal au moins et l'autre extension (52) comportant un autre orifice distal pour recevoir l'endoprothèse dilatable d'artère iliaque respective (4, 5), chacune de celles-ci comportant un stent dilatable jusqu'à un diamètre tel que chaque endoprothèse dilatable d'artère iliaque (4, 5) ait une superficie de section suffisamment supérieure à la ou aux superficie(s) de section desdits orifices distaux quand les extensions courtes ont été dilatées, pour former des joints d'étanchéité efficaces.

4. Dispositif constitué d'une endoprothèse (1, 4, 5) selon l'une quelconque des revendications précédentes, dans lequel le ou les stent(s) de ladite endoprothèse additionnelle (50) est ou sont équipé(s) de pointes, de barbillons ou de crochets pour faciliter la fixation au niveau de l'aorte.

5. Dispositif constitué d'une endoprothèse (1, 4, 5) selon l'une quelconque des revendications précédentes, dans lequel le renforcement au niveau de la deuxième extrémité de l'endoprothèse additionnelle (50) est fourni par des plis (50) formés dans une enveloppe pliée à l'intérieur (48) de façon à produire une plus grande épaisseur de matériau d'enveloppe.

6. Dispositif constitué d'une endoprothèse (1, 4, 5) selon l'une quelconque des revendications précédentes, dans lequel l'enveloppe externe (48) comporte une extension proximale sur l'élément externe de chaque joint d'étanchéité, ladite extension étant conçue de façon à être repliée par-dessus l'extrémité proximale de l'élément externe et à pénétrer dans l'extrémité proximale de l'élément interne de chaque joint d'étanchéité, cette enveloppe repliée fournissant un dispositif d'étanchéité additionnel.
